# EUROPÄISCHE PATENTSCHRIFT

(11) **EP 2 987 524 B1**
(45) Veröffentlichungstag und Bekanntmachung des Hinweises auf die Patenterteilung: **23.05.2018**
(21) Anmeldenummer: 15186226.5
(22) Anmeldetag: 02.02.2007
(51) Int. Cl.: A61M 15/00, A61M 16/04, A61M 11/00, A61M 16/00, A61M 11/02, A61M 16/08, A61M 16/10, A61M 16/14, A61M 16/16

(54) **INHALATIONSTHERAPIEVORRICHTUNG FÜR DIE ANWENDUNG BEI FRÜHGEBORENEN UND KLEINKINDERN**
INHALATION THERAPY DEVICE FOR USE WITH PREMATURE BABIES AND INFANTS
DISPOSITIF DE THERAPIE PAR INHALATION A UTILISER PAR DES PREMATURES ET DES ENFANTS EN BAS AGE

(30) Priorität: 10.02.2006 DE 102006006183
(43) Veröffentlichungstag der Anmeldung: 24.02.2016
(62) Teilanmeldung aus: 07002327.0
(73) Patentinhaber: PARI Pharma GmbH, 82319 Starnberg (DE)
(72) Erfinder: Minocchieri, Stefan, 8180 Bülach (CH); Gallem, Thomas, 81371 München (DE); Vogelmann, Martina, 82407 Wielenbach (DE)
(74) Vertreter: Hoffmann Eitle

(56) Entgegenhaltungen:
- WO-A2-2005/048982
- WO-A2-2005/102431
- US-A- 5 443 059
- US-A- 6 014 972
- US-A1- 2005 087 189
- US-B1- 6 729 334

## Beschreibung

Die vorliegende Erfindung betrifft eine Inhalationstherapievorrichtung für die Anwendung bei Frühgeborenen und Kleinkindern.

Frühgeborene Kinder unter 34 Schwangerschaftswochen leiden an einem Surfactant-Mangelsyndrom. Synonyme für diese Krankheit sind: HMK (Hyaline-Membrankrankheit), Atemnotsyndrom des Frühgeborenen, IRDS (Infant Respiratory Distress Syndrome). Die Surfactant-Substitutionstherapie ist bereits gut etabliert und gehört zu den Standardtherapieverfahren in der Neonatologie (Frühgeborenen- und Neugeborenenmedizin). Um den Umfang des Einsatzbereichs einer erfindungsgemäßen Inhalationstherapievorrichtung anzudeuten, sei darauf hingewiesen, dass in der Schweiz pro Jahr ca. 550 Kinder vor Erreichen der 35. Schwangerschaftswoche geboren werden und damit potenziell eine unreife Lunge aufweisen, bei der eine Surfactant-Substitutionstherapie angezeigt ist. In anderen Ländern, beispielsweise Deutschland, kann mit einer zehnfach höheren Zahl frühgeborener Kinder gerechnet werden.

Die Surfactant-Substitutionstherapie erfolgt, während sich die Frühgeborene/Kleinkinder in sogenannten Brutkästen befinden, dass heißt in einer Umgebung mit kontrollierter Temperatur und Luftfeuchte, da die Frühgeborenen ihre Körpertemperatur noch nicht selbst aufrecht erhalten können. Das Surfactant wird über einen Tubus direkt in die Trachea in flüssiger Form instilliert. Die Intubation an sich birgt verschiedene Risiken, zum Beispiel Verletzung der Glottis oder der Luftröhre, Pneumothorax etc. Überdies kann die maschinelle Beatmung, die in der Regel mit der Instillation einhergeht, zu zusätzlichen Schädigungen der Lunge führen. Viele der Frühgeborenen/Kleinkinder haben jedoch einen ausreichenden eigenen Atemantrieb und müssten vor diesem Hintergrund nicht intubiert werden. Um jedoch das Surfactant in der Lunge zu deponieren, ist die Intubation das Mittel der Wahl für die Instillation des Surfactants.

Während die Surfactant-Substitutionstherapie intensiv untersucht wurde und bereits umfangreich eingesetzt wird, ist die Verneblung des Surfactants problembehaftet, da das Surfactant regelmäßig eine geringe Oberflächenspannung, eine für die Vernebelung ungünstige Viskosität und eine Neigung zur Schaumbildung aufweist. Die physikalischen Eigenschaften des Surfactants haben dazu geführt, dass an eine Verneblung und die Applikation des Surfactants in Form eines Aerosols praktisch nicht gedacht wurde. Zudem ist ein Surfactant in der Regel sehr teuer, so dass die bei Aerosoltherapie oftmals zu beobachtenden hohen Depositionsverluste dazu geführt haben, dass diese Art der Applikation eines Surfactants nicht weiter untersucht wurde.

Eine Inhalationstherapievorrichtung mit den Merkmalen im Oberbegriff von Anspruch 1 ist aus der US 5,442,059 B1 bekannt. Die US 5,442,059 B1 offenbart ebenso wie die WO 2005/102431 A2 und die WO 2005/048982 A2 eine Ansteuerung des Piezo-Schwingers in Abhängigkeit des Atemzyklus. Ferner ist aus der US 6,729,334 B1 die Optimierung der Aerosolerzeugung durch Einstellung der Amplitude und Frequenz des Aerosolerzeugers bekannt.

Vor diesem Hintergrund strebt die vorliegende Erfindung an, einen Weg aufzuzeigen, wie Surfactant bei Frühgeborenen und Kleinkindern im Rahmen einer Aerosoltherapie appliziert werden kann.

Dieses Ziel wird erreicht durch eine Inhalationstherapievorrichtung mit den Merkmalen in Anspruch 1.

Im Folgenden wird die Erfindung anhand von Ausführungsbeispielen genauer erläutert. Dabei wird auf die Figuren Bezug genommen, in denen zeigt:
- Figur 1: ein erstes Ausführungsbeispiel einer erfindungsgemäßen Inhalationstherapievorrichtung, mit den grundlegenden Komponenten;
- Figur 1a: eine vergrößerte Ansicht eines in besonderer Weise ausgestalteten Teils des Ausführungsbeispiels gemäß Figur 1;
- Figur 2: ein zweites Ausführungsbeispiel einer erfindungsgemäßen Inhalationstherapievorrichtung mit einer Atemluftheizeinrichtung;
- Figur 3: ein drittes Ausführungsbeispiel einer erfindungsgemäßen Inhalationstherapievorrichtung mit einer Atemluftbefeuchtungseinrichtung;
- Figur 4: ein viertes Ausführungsbeispiel einer erfindungsgemäßen Inhalationstherapievorrichtung mit einer Atemluftstrompulsationseinrichtung; und
- Figur 5: ein fünftes Ausführungsbeispiel einer erfindungsgemäßen Inhalationstherapievorrichtung mit mehreren Zusatzeinrichtungen und einer Steuereinrichtung.

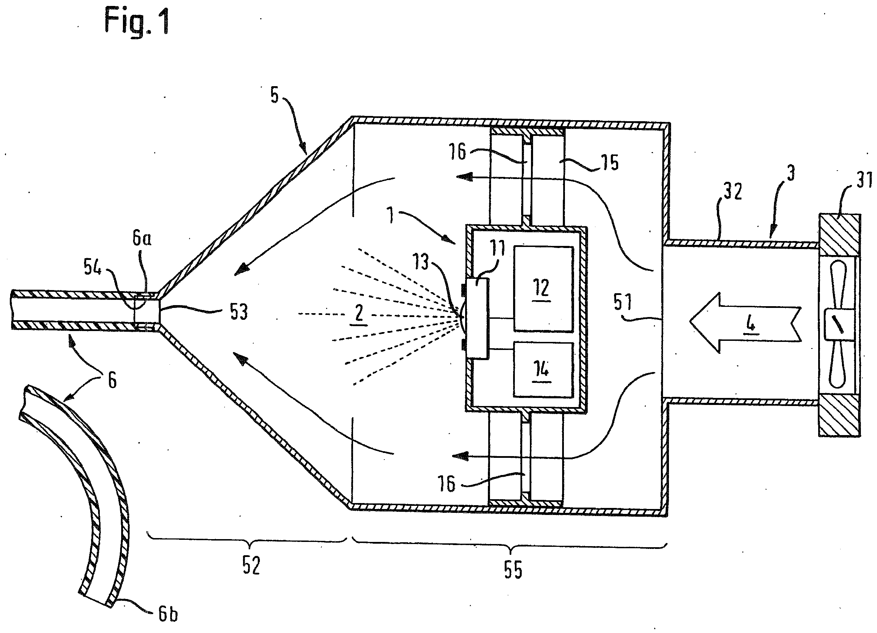

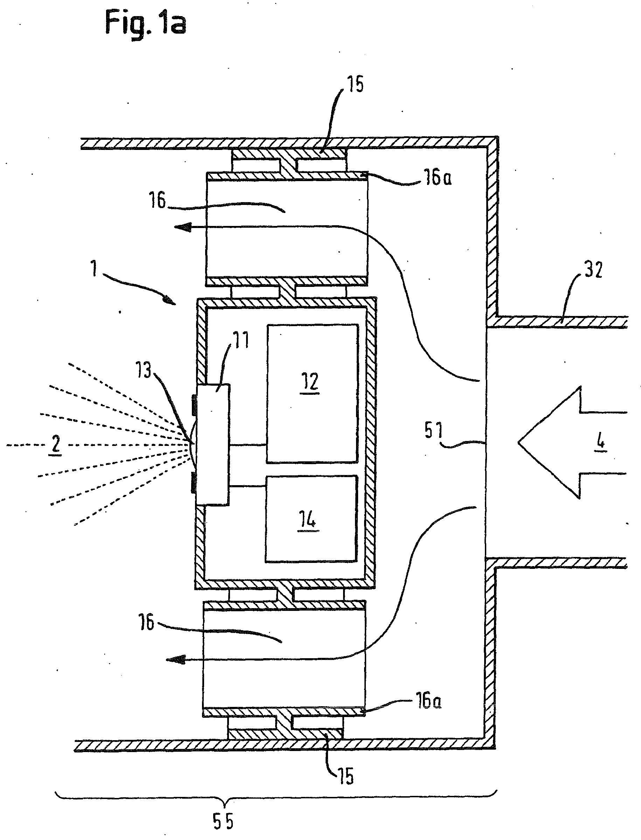

Bei der in Figur 1 gezeigten erfindungsgemäßen Inhalationstherapievorrichtung ist eine Aerosolerzeugungseinrichtung 1 für die Vernebelung eines Fluids und die Bereitstellung von Flüssigkeitströpfchen 2 vorgesehen. Die schematisch dargestellte Aerosolerzeugungseinrichtung 1 umfasst in der gezeigten Ausgestaltung einen Aerosolgenerator 11, dem eine Flüssigkeit zugeführt wird, die in einem Reservoir 12 bevorratet wird. In der gezeigten Ausgestaltung umfasst der Aerosolgenerator 11 eine Membran 13, mit deren Hilfe die aus dem Reservoir zugeführte Flüssigkeit vernebelt wird, so dass der Aerosolgenerator 11 eine definierte Menge von Flüssigkeitströpfchen 2 abgibt. Der Aerosolgenerator 11 wird von einer Aerosolerzeugungssteuerung 14 der Aerosolerzeugungseinrichtung 1 gesteuert.

Vor dem Hintergrund des Einsatzes bei Frühgeborenen und Kleinkindern liegt die Größe der Flüssigkeitströpfchen (MMD) bei einer erfindungsgemäßen Inhalationstherapievorrichtung zwischen 1,5 und 3 µm. Die Einhaltung dieser Vorgaben ist mit besonders hoher Genauigkeit bei einer Aerosolerzeugungseinrichtung 1 möglich, die einen zuvor bereits angesprochenen Aerosolgenerator 11 mit einer Membran 13 für die Erzeugung der Flüssigkeitströpfchen aufweist. Die in Figur 1 gezeigte erfindungsgemäße Inhalationstherapievorrichtung umfasst ferner eine Einrichtung 3 zur Erzeugung einer Atemluftströmung 4. Dazu umfasst die Atemluftstromerzeugungseinrichtung 3 beispielsweise einen Ventilator 31, der den Atemluftstrom 4 erzeugt und in eine Zuführleitung 32 hineinfördert. Durch eine entsprechende Steuerung der Atemluftstromerzeugungseinrichtung 3 sind Druck und Fluss der Atemluftströmung 4 an die spezifische Therapiesituation anpassbar.

Bei Frühgeborenen und Kleinkindern sind ein Druck von maximal 4 bis 7 mbar und ein Atemvolumen von etwa 5 ml pro kg Körpergewicht als geeignete Vorgabe anzusetzen. Bei diesen Vorgaben erfolgt die Ventilation der Frühgeborenen/Kleinkinder vor dem Hintergrund der eigenen Atmung entsprechend dem CPAP-Prinzip (Continuous Positive Airway Pressure). Voraussetzung für die Anwendung einer CPAP-Ventilation ist stets die Fähigkeit des Patienten zur Atmung, wobei aber bei Frühgeborenen und Kleinkindern durch den CPAP-Überdruck vorteilhafterweise erreicht wird, dass die Lungen etwas vorgebläht werden und ein Kollabieren bereits belüfteter Alveolen verhindert wird. Andere Verfahren, wie zum Beispiel nach dem BIPAP-Prinzip (Biphasic Positive Airway Pressure) sind ebenfalls anwendbar. Die anwendbaren Drücke richten sich nach den konkreten Gegebenheiten und können Werte von 10 mbar (CPAP) und 15 mbar (BIPAP) erreichen und sogar überschreiten. Die Figur 1 gezeigte erfindungsgemäße Inhalationstherapievorrichtung weist ferner eine Vernebelungskammer 5 auf, in die sowohl die Flüssigkeitströpfchen 2, die von der Aerosolerzeugungseinrichtung 1 erzeugt werden, als auch die Atemluftströmung 4, die von der Atemluftstromerzeugungseinrichtung 3 erzeugt wird, zugeführt werden. Bei dem in Figur 1 gezeigten Ausführungsbeispiel geschieht dies im Hinblick auf die Flüssigkeitströpfchen 2 dadurch, dass die Aerosolerzeugungseinrichtung 1 in der Vernebelungskammer 5 angeordnet ist, so dass die Abgabe der Flüssigkeitströpfchen 2 unmittelbar in der Vernebelungskammer 5 erfolgt. Diese Anordnung der Aerosolerzeugungseinrichtung 1 führt auch zu einer Gestaltung des erfindungsgemäßen Inhalationstherapiesystems, bei dem Totvolumina der Vernebelungskammer 5 auf ein Minimum reduziert werden können.

Die Zuführung der Atemluftströmung 4 erfolgt durch eine Atemluftzuführöffnung 51 der Vernebelungskammer 5, an der die Zuführleitung 32 der Atemluftstromerzeugungseinrichtung 3 angeordnet ist. Die Zuführung erfolgt vorzugsweise derart, dass sich beim Eintreten der Atemluft 4 in die Vernebelungskammer 5 eine verwirbelte Strömung ausbildet. Auf diese Weise wird sichergestellt, dass nur minimale Toträume der Vernebelungskammer 5 in der Nähe der Atemluftzuführöffnung 51 unter Umständen nicht durchströmt werden, wodurch eine Versorgung des Frühgeborenen/Neugeborenen mit sauerstoffhaltiger Frischluft bestmöglich gewährleistet wird. In der Regel wird aber die verwirbelte Strömung eine vollständige oder weitgehend vollständige Durchströmung der Vernebelungskammer 5 gewährleisten. Wie weiter unten noch erläutert wird, sind in einer bevorzugten Ausgestaltung der Erfindung Mittel vorgesehen, die die verwirbelte Strömung in eine weitgehend gerichtete Strömung überführen.

Die Flüssigkeitströpfchen 2 und die Atemluft 4 vermischen sich in der Verneblungskammer 5 und gelangen aufgrund der durch die Atemluft 4 vorgegebenen Strömung, wie Figur 1 zeigt, in einen sich verjüngenden Bereich 52 der Vernebelungskammer 5, der in eine Austrittsöffnung 53 mündet. Durch die Öffnung 53 der Vernebelungskammer 5 kann das Flüssigkeitströpfchen/Atemluft-Gemisch, das durch Zuführen der Flüssigkeitströpfchen 2 und der Atemluft 4 in die Vernebelungskammer 5 gebildet wurde, aus der Vernebelungskammer 5 austreten.

Erfindungsgemäß weist das in Figur 1 gezeigte Ausführungsbeispiel eine schlauchförmige Intubierungseinrichtung 6 auf, deren erstes Ende 6a für den Anschluss an die Austrittsöffnung 53 der Vernebelungskammer 5 ausgebildet ist. Vorzugsweise ist das erste Ende 6a der Intubierungseinrichtung 6 auf einen Stutzen 54 der Vernebelungskammer 5 aufsteckbar, der an der Austrittsöffnung 53 vorgesehen ist. Durch die Anbringung der schlauchförmigen Intubierungseinrichtung 6 gemäß der Erfindung tritt das Flüssigkeitströpfchen/Atemluft-Gemisch aus der Vernebelungskammer 5 durch die Austrittsöffnung 53 in die schlauchförmige Intubierungseinrichtung 6 ein, durchströmt diese und gelangt zum zweiten Ende 6b der schlauchförmigen Intubierungseinrichtung 6.

Um einen weitgehend depositionsfreien Transport des Flüssigkeitströpfchen/Atemluft-Gemisches durch die Intubierungseinrichtung zu gewährleisten, wird zweckdienlicherweise ein Schlauch mit 2 bis 3,5 mm Innendurchmesser eingesetzt. Ebenfalls zur Minimierung von Depositionsverlusten sollte die Gesamtlänge der schlauchförmigen Intubierungseinrichtung 50 cm nicht übersteigen. Überraschenderweise stellen sich bei Einhaltung dieser Vorgaben sehr gute Ergebnisse ein, die so angesichts der vergleichsweise klein erscheinenden Passagen für das Aerosol nicht erwartet werden konnten. Dies gilt umsomehr für ein Surfactant als zu vernebelnde Flüssigkeit, deren physikalische Eigenschaften nicht erahnen ließen, dass bei Einhaltung bestimmter Vorgaben und geeigneter Vernebelung eine Aerosol-Applikation von Surfactant bei Frühgeborenen und Kleinkindern auf dem Wege der Inhalation möglich ist.

Das zweite Ende der Intubierungseinrichtung 6 ist für eine endotracheale/endopharyngeale Intubation ausgestaltet, wobei erfindungsgemäß die Ausgestaltung vorteilhaft derart erfolgt, dass das zweite Ende bei einer orotrachealen Intubation durch den Mund hinter den Stimmlippen eines Patienten und bei einer nasophayngealen Intubation durch die Nase hinter dem Nasenraum im Rachen eines Patienten positionierbar ist. Durch die erfindungsgemäße Positionierbarkeit des zweiten Endes 6b der schlauchförmigen Intubierungseinrichtung 6 wird sichergestellt, dass das durch die Intubierungseinrichtung geförderte Flüssigkeitströpfchen/Atemluft-Gemisch hinter die jeweils stark filternden Bereiche des Atemwegs des Patienten gelangt. Im Fall einer Applikation durch die Nase ist es erforderlich, den Nasenbereich zu überbrücken und das Flüssigkeitströpfchen/Atemluft-Gemisch in den Rachen des Patienten abzugeben, wohingegen bei einer Applikation über den Mund die Abgabe des Flüssigkeitströpfchen/Atemluft-Gemisches vorzugsweise hinter der Glottis erfolgt. Bei der erfindungsgemäßen Ausgestaltung des Intubationsendes 6b liegt das Hauptaugenmerk demzufolge auf der Länge dieses Bereichs, da die Länge des zweiten Endes 6b der Intubierungseinrichtung 6 festlegt, an welchem Punkt der Patientenatemwege die Abgabe des Flüssigkeitströpfchen/Atemluft-Gemisches (des Aerosols) erfolgt. Bei Frühgeborenen und Kleinkindern ist eine Länge von ca. 15 cm zweckdienlich.

Im Zusammenwirken der einzelnen Komponenten der in Figur 1 gezeigten erfindungsgemäßen Inhalationstherapievorrichtung wird erreicht, dass die Flüssigkeitströpfchen 2 und die Atemluftströmung 4 in der Vernebelungskammer 5 zusammengeführt werden, sich dort vermischen und als Flüssigkeitströpfchen/Atemluft-Gemisch, das heißt als Aerosol, über den verjüngten Bereich 52 der Vernebelungskammer in die Intubierungseinrichtung 6 gefördert werden, um erst hinter den stark filternden Atemwegsbereichen des Patienten aus der Intubierungseinrichtung abgegeben zu werden. Damit erzielt die erfindungsgemäße Inhalationstherapievorrichtung eine besonders wirksame Applikation der vernebelten Flüssigkeit, so dass sich für die erfindungsgemäße Inhalationstherapievorrichtung Anwendungsgebiete erschließen, die bei herkömmlichen Vorrichtungen aufgrund mangelhafter Applikationsgenauigkeit und -effektivität erreichbar waren.

So ist, wie eingangs angesprochen, mit Hilfe einer erfindungsgemäßen Inhalationstherapievorrichtung die Verabreichung von Surfactant bei Frühgeborenen und Kleinkindern möglich. Surfactant reduziert die Oberflächenspannung in den Alveolen und erleichtert somit den Kindern die Atemarbeit, was zu einer verbesserten Sauerstoffaufnahme führt. Die positive Wirkung einer Behandlung mit Surfactant ist in der Literatur vielfach beschrieben. Unbekannt ist jedoch die Applikation eines Surfactant-Aerosols, das mit Hilfe der erfindungsgemäßen Inhalationstherapievorrichtung appliziert werden kann. Diese Möglichkeit wird durch die Erfindung geschaffen, da das mehrere Aspekte umfassende Gesamtkonzept der erfindungsgemäßen Inhalationstherapievorrichtung dazu führt, dass eine Therapievorrichtung zur Verfügung steht, die die Applikation eines Surfactants auf diese Weise ermöglicht.

Zu dem in Figur 1 gezeigten Ausführungsbeispiel ist anzumerken, dass die Anordnung der Aerosolerzeugungseinrichtung 1 im Inneren der Vernebelungskammer 5 besonders vorteilhaft ist, da auf diese Weise eine unerwünschte Deposition der Flüssigkeitströpfchen in der Vernebelungskammer 5 minimiert wird. Die Anordnung trägt außerdem zur Minimierung von Totraumvolumina in der Vernebelungskammer 5 bei. Überdies kann die Anordnung des Aerosolgenerators 11 der Aerosolerzeugungseinrichtung 1 so erfolgen, dass sie bezogen auf den verjüngten Bereich 52 der Vernebelungskammer in geeigneter Weise ausgerichtet ist. Bei einer grundsätzlich rotationssymmetrischen Gestaltung der erfindungsgemäßen Inhalationstherapievorrichtung, wie sie in Figur 1 angedeutet ist, bedeutet dies, dass der Aerosolgenerator 11 der Aerosolerzeugungseinrichtung 1 auf der Rotationsachse derart angeordnet ist, dass die Flüssigkeitströpfchen sich entlang der Rotationsachse auf den sich verjüngenden Bereich 52 zu bewegen. Damit ist die erfindungsgemäße Vorrichtung auch in beliebigen Lagen in Bezug auf die Rotationsachse betreibbar. Auch die Zuführung der Atemluft 4 erfolgt dann entlang der Rotationsachse, so dass sich eine um die Aerosolerzeugungseinrichtung herum laufende Atemluftströmung einstellt, die in Figur 1 durch entsprechende Pfeile angedeutet ist. Damit die Atemluft 4 die Aerosolerzeugungseinrichtung 1 umströmen kann, weist eine Halterungseinrichtung 15 der Aerosolerzeugungseinrichtung 1 Durchtrittsöffnungen 16 auf, damit die Atemluft 4 die Aerosolerzeugungseinrichtung 1 in der Vernebelungskammer 5 praktisch ungehindert umströmen kann. Jedoch bewirken die Durchtrittsöffnungen 16 in vorteilhafter Weise, dass eine sich nach dem Eintritt durch die Atemlufzuführöffnung verwirbelt ausbildende Atemluftströmung in eine weitgehend laminare Strömung umgewandelt wird, die den von Trägheitskräften weitestgehend unbeeinflussten Transport der zuströmenden Flüssigkeitströpfchen begünstigt. Im übrigen weist die Halterungseinrichtung 15 aber eine ausreichende Anzahl von radial verlaufenden Stützelementen auf, so dass die Halterungseinrichtung 15 die Aerosolerzeugungseinrichtung 1 in dem dafür vorgesehenen Bereich 55 der Vernebelungskammer 5 fixiert.

Um die Umwandlung der Atemluftströmung in eine weitestgehend laminare Strömung zu unterstützen, können die Durchtrittsöffnungen 16 mit Atemluftführungen 16a ausgestattet werden, wie Figur 1a zeigt. Vorteilhafterweise sind die Atemluftführungen kurze rohrförmige Elemente 16a, die beispielsweise einen kreisförmigen Querschnitt aufweisen.

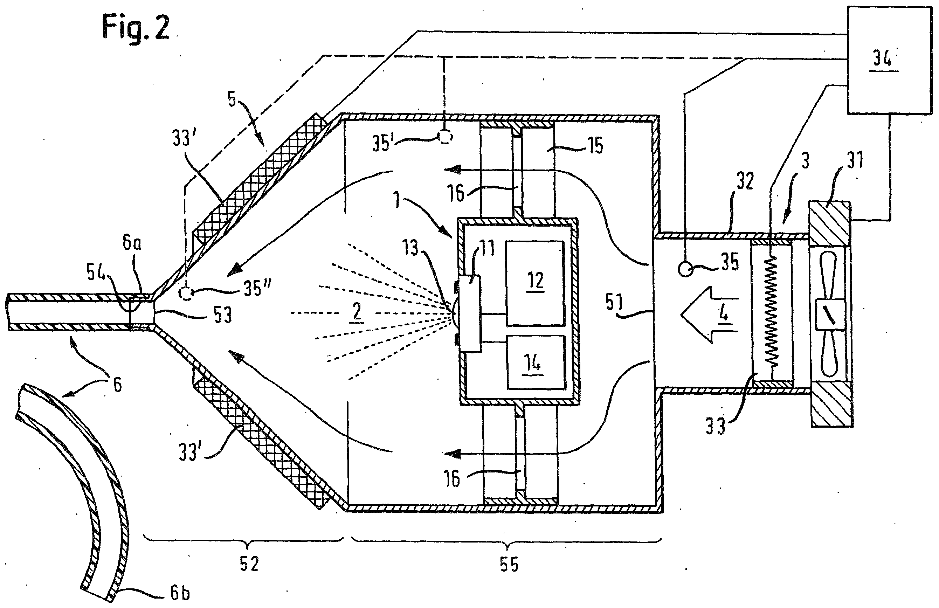

Figur 2 zeigt ein weiteres Ausführungsbeispiel einer erfindungsgemäßen Inhalationstherapievorrichtung, das hinsichtlich der Grundkomponenten mit dem ersten Ausführungsbeispiel übereinstimmt. Insofern wird auf die vorangegangene Beschreibung Bezug genommen; Figur 2 weist demzufolge auch dieselben Bezugszeichen auf, die in Figur 1 verwendet wurden.

Bei dem zweiten Ausführungsbeispiel ist, wie in Figur 2 gezeigt, eine Atemluftheizvorrichtung 33 vorgesehen, die die Atemluft 4 erwärmt, die von der Atemluftstromerzeugungseinrichtung 3 in die Vernebelungskammer 5 gefördert wird. Für die Regelung der Atemlufttemperatur ist eine Atemluftsteuereinrichtung 34 vorgesehen, die die Atemluftheizeinrichtung 33 auf der Basis eines Messsignals steuert, das von einem Temperatursensor 35 bereitgestellt wird. Der Sensor 35 ist in der Strömung der erwärmten Atemluft 4 angeordnet, beispielsweise in der Zuführleitung 32 der Atemluftstromerzeugungseinrichtung 3. Alternativ kann, wie in Figur 2 gezeigt, der Atemluftsensor 35', 35'' auch an einer geeigneten Stelle in oder an der Vernebelungskammer 5 angeordnet sein. In einer besonders bevorzugten Ausgestaltung erfolgt die Temperaturmessung durch einen Sensor 35'' in der Nähe der Austrittsöffnung 53 der Vernebelungskammer 5. Jedoch können auch zwei oder mehr Sensoren eingesetzt werden, um beispielsweise die Temperatur der Atemluft im Bereich der Zuführleitung 32 einerseits und im Bereich der Austrittsöffnung 53 andererseits zu erfassen und bei der Steuerung der Atemluftheizeinrichtung 33 durch die Atemluftsteuereinrichtung 34 zu berücksichtigen.

Die Atemluftheizeinrichtung 33 wird vorzugsweise durch die Atemluftsteuereinrichtung 34 derart angesteuert, dass die über die Intubiereinrichtung dem Patienten zugeführte Atemluft auf 35°C bis 37°C erwärmt wird. Die Atemluftsteuereinrichtung 34 regelt die Temperatur der Atemluft unabhängig von den äußeren Bedingungen in einem engen Bereich. Dabei kann die Atemluftsteuereinrichtung 34 mit der Luftfördereinrichtung 31 verbunden sein und deren Ventilator unter Berücksichtigung der Messwerte steuern, die von den Messsensoren 35, 35' und/oder 35'' zur Atemluftsteuereinrichtung 34 geliefert werden.

Schließlich kann eine Verbindung zwischen der Aerosolerzeugungseinrichtung 1, genauer deren Steuereinrichtung 14 und der Atemluftsteuereinrichtung 34 zweckdienlich sein, damit der Beginn der Vernebelung der Flüssigkeit bei der Ansteuerung der Atemluftheizeinrichtung 33 berücksichtigt wird. Denn durch die Erzeugung der Flüssigkeitströpfchen 2 in der Vernebelungskammer 5 tritt eine Abkühlung der in der Vernebelungskammer 5 vorhandenen Atemluft auf, die sich mit den Flüssigkeitströpfchen durchmischt, da die Flüssigkeitströpfchen durch die Atemluft abgetrocknet werden. Dieser Abtrocknungseffekt ist grundsätzlich wünschenswert, da dadurch Einfluss genommen werden kann auf die Größe der Flüssigkeitströpfchen, so dass eine genauere Einhaltung der Vorgaben (siehe oben) möglich ist. Durch eine entsprechende Steuerung der Heizeinrichtung 33 kann somit letztlich die Temperatur und die Tröpfchengröße (durch kontrollierte Abtrocknung) des Aerosols festgelegt werden, insbesondere dann, wenn ein Sensor 35'' in der Nähe der Austrittsöffnung 53 der Vernebelungskammer 5 eingesetzt wird. Unterstützend kann eine Erwärmungseinrichtung für die in dem Aerosolerzeuger bevorratete Flüssigkeit vorgesehen werden, die die bevorratete Flüssigkeit erwärmt. Vorzugsweise wird die Erwärmungseinrichtung von der Steuereinrichtung 14 gesteuert.

Als Alternative oder zusätzlich zu der in Figur 2 gezeigten Atemluftheizeinrichtung 33 kann in einer weiteren Ausgestaltung der erfindungsgemäßen Inhalationstherapievorrichtung eine Aerosolheizeinrichtung 33' vorgesehen werden, die vorzugsweise an der Vernebelungskammer 5 angeordnet ist, wie auch Figur 2 zeigt. Die Aerosolheizeinrichtung 33' erwärmt das Aerosol durch IR-Strahlung. Auch die Aerosolheizeinrichtung 33' ist vorteilhafterweise an die Atemluftsteuereinrichtung 34 angeschlossen und wird wie zuvor für die Heizeinrichtung 33 beschrieben in die Steuerung einbezogen. Auf die vorstehende Beschreibung wird insofern Bezug genommen.

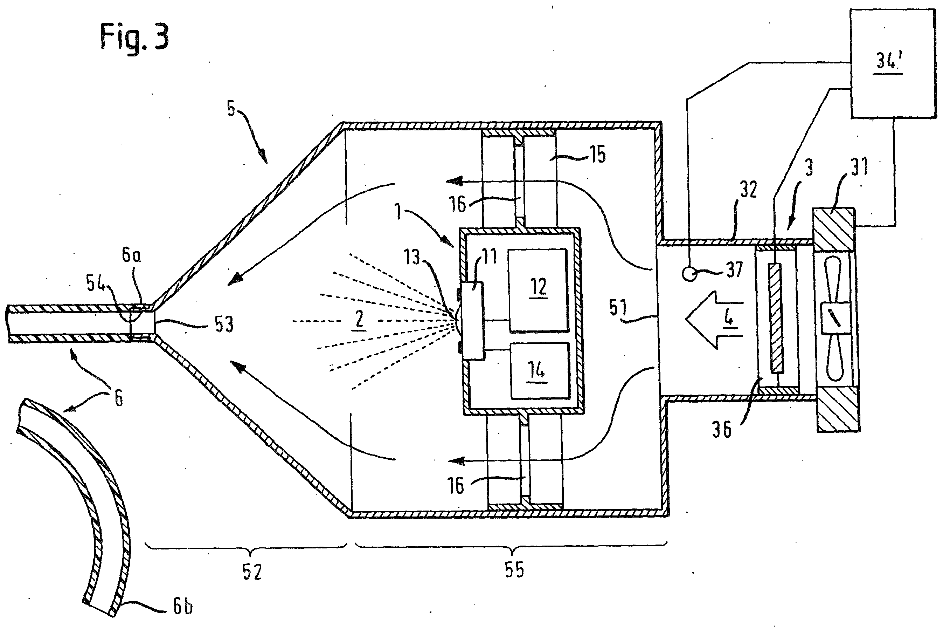

Figur 3 zeigt ein weiteres Ausführungsbeispiel einer erfindungsgemäßen Inhalationstherapievorrichtung. Ebenso wie bei Figur 2 stimmt der Aufbau des Ausführungsbeispiels gemäß Figur 3 im Wesentlichen mit dem Aufbau des ersten Ausführungsbeispiels überein. Dementsprechend sind die Bezugszeichen aus Figur 1 auch in Figur 3 vorhanden.

In Abwandlung des ersten Ausführungsbeispiels umfasst die erfindungsgemäße Inhalationstherapievorrichtung gemäß Figur 3 eine Atemluftbefeuchtungseinrichtung 36, die in Bezug auf die Atemluftströmung 4 derart angeordnet ist, dass sie die von der Atemluftströmerzeugungseinrichtung 3 geförderte Atemluft befeuchten kann. Angesteuert wird die Atemluftbefeuchtungseinrichtung 36 durch die Atemluftsteuereinrichtung 34', die vorzugsweise ein Messsignal von einem Feuchtesensor 37 erhält, der in dem befeuchteten Atemluftstrom 4 angeordnet ist und mit der Atemluftsteuereinrichtung 34' verbunden ist. Optional ist die Atemluftsteuereinrichtung 34', ebenso wie die im Zusammenhang mit Figur 2 beschriebene Atemluftsteuereinrichtung 34, mit der Atemluftfördereinrichtung 31 verbunden, um den Ventilator zu steuern. Ferner steht vorzugsweise die Atemluftsteuereinrichtung 34' in Verbindung mit der Steuereinrichtung 14 der Aerosolerzeugungseinrichtung 1, um die Befeuchtungseinrichtung 36 in den Phasen anzusteuern, in denen keine Aerosolerzeugung, d.h. keine Abgabe von Flüssigkeitströpfchen 2 durch die Aerosolerzeugungseinrichtung 1 stattfindet. In diesen Phasen ist das Anfeuchten der Atemluft besonders vorteilhaft, während eine zu feuchte Atemluft während der Medikamentengabe, d.h. bei angesteuertem Aerosolgenerator 11 verhindert, dass die Tröpfchen 2 durch die zugeführte Atemluft 4 weiter abgetrocknet werden.

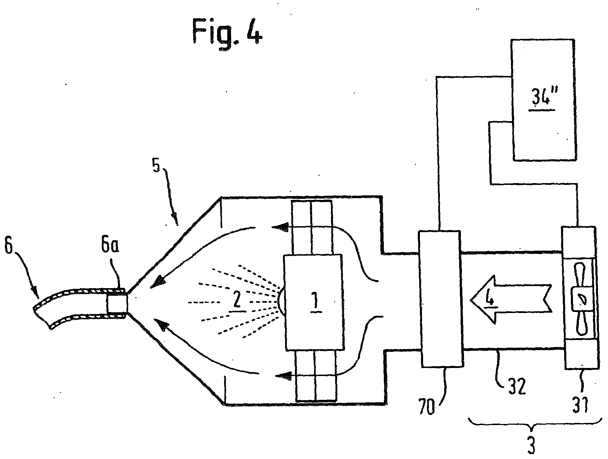

Figur 4 zeigt ein viertes Ausführungsbeispiel einer erfindungsgemäßen Inhalationstherapievorrichtung, jedoch in einer vereinfachten Darstellung, um die Komplexität zu reduzieren. Dennoch umfasst auch das vierte Ausführungsbeispiel vorzugsweise die Komponenten, die zuvor insbesondere im Zusammenhang mit dem ersten Ausführungsbeispiel ausführlich geschildert wurden. Insofern wird auf die Beschreibung des ersten bis dritten Ausführungsbeispiels Bezug genommen, ohne dass aber sämtliche Komponenten in Figur 4 erneut gezeigt und mit Bezugszeichen versehen sind.
Im Vordergrund des vierten Ausführungsbeispiels steht die in Figur 4 gezeigte Atemluftpulsationseinrichtung 70, die auf die Atemluftströmung 4 einwirkt, um der Atemzuluft zur Verneblerkammer 5 Druckschwingungen zu überlagern. Auf diese Weise wird die Atemluft, die zusammen mit den Flüssigkeitströpfchen aus der Verneblerkammer 5 über die Intubierungseinrichtung 6 dem Frühgeborenen/Kleinkind zugeführt wird, mit einer überlagerten Vibration (Druckschwankungen/-schwingungen) zugeführt, was zur Rekrutierung zusätzlicher Lungenbereiche führen kann. Rekrutierung bedeutet in diesem Zusammenhang, dass eine Lungenregion, welche vorher nicht am Gasaustausch teilgenommen hat, aktiviert wird.

Die Pulsationseinrichtung 70, die in Figur 4 schematisch gezeigt ist, kann auf verschiedene Weise realisiert werden, beispielsweise durch ein steuerbares oder selbsttätiges Ventilsystem oder die Aufbringung eines Wechseldrucks, der von einem Kolbenkompressor hervorgerufen wird. Gesteuert wird die Pulsationseinrichtung 70 durch eine Atemluftsteuereinrichtung 34'', die vorzugsweise auch mit der Fördereinrichtung 31 der Atemluftstromerzeugungseinrichtung 3 verbunden ist und diese steuert. Offensichtlich kann die Atemluftsteuereinrichtung 34'' gemäß dem vierten Ausführungsbeispiel auch mit der Steuereinrichtung 14 der Aerosolerzeugungseinrichtung 1 verbunden sein, um eine Steuerung der Pulsationseinrichtung 70 in Abhängigkeit von bzw. unter Berücksichtigung der Erzeugung des Aerosols 2 durchzuführen.

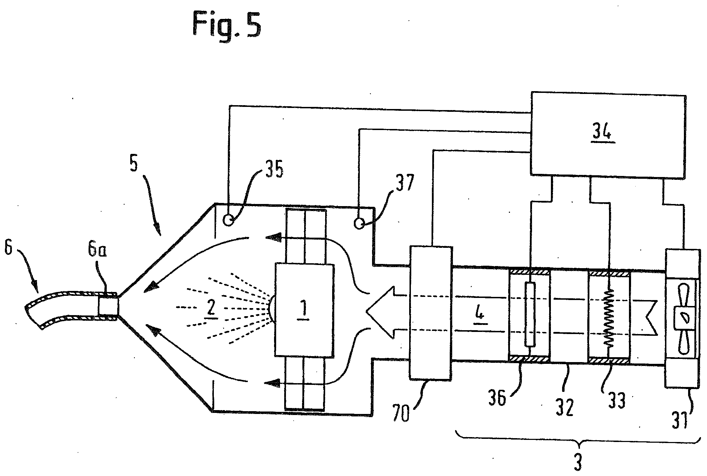

Figur 5 zeigt ein fünftes Ausführungsbeispiel der erfindungsgemäßen Inhalationstherapievorrichtung, bei dem die aus den Figuren 2 bis 4 ersichtlichen zusätzlichen Komponenten in einer Vorrichtung zusammengefasst sind. Dadurch soll beispielhaft zum Ausdruck gebracht werden, dass die zusätzlich zu den Grundkomponenten vorhandenen Zusatzeinrichtungen auch in unterschiedlicher Kombination gemeinsam eingesetzt werden können.

Diesem Gedanken folgend zeigt Figur 5 ein Ausführungsbeispiel, bei dem neben den Grundkomponenten, die im Zusammenhang mit Figur 1 ausführlich beschrieben wurden und die aus diesem Grunde hier nicht erneut vollständig erläutert und in Figur 5 gezeigt werden, gemäß Figur 5 die Atemluftheizeinrichtung 33, die Atemluftbefeuchtungseinrichtung 36 und die Pulsationseinrichtung 70 gemeinsam vorgesehen sind. Die Steuerung der Atemluftheizeinrichtung 33, der Atemluftbefeuchtungseinrichtung 36 und der Pulsationseinrichtung 70 übernimmt die Atemluftsteuereinrichtung 34, die dazu mit den Einrichtungen 33, 36 und 70 verbunden ist. Ferner ist vorzugsweise die Atemluftsteuereinrichtung 34 mit der Fördereinrichtung 31 der Atemluftstromerzeugungseinrichtung 3 verbunden. Messsignale erhält die Atemluftsteuereinrichtung 34 von dem(n) Temperatursensor(en) 35, 35', 35'' und dem Feuchtesensor 37, die beide in Figur 5 schematisch dargestellt sind. Nicht in Figur 5 dargestellt ist eine Verbindung zwischen der Atemluftsteuereinrichtung 34 und der in der Aerosolerzeugungseinrichtung 1 vorhandenen Steuereinrichtung 14 (vgl. Figuren 1 bis 3), die zweckdienlicherweise vorgesehen ist, um die Ansteuerung der Einrichtungen 33, 36 und 70 sowie 31 unter Berücksichtigung der Aerosolerzeugung durch die Aerosolerzeugungseinrichtung 1 durchzuführen.

Wie sich aus allen Figuren und den darin gezeigten Ausführungsbeispielen ergibt, umfasst die erfindungsgemäße Inhalationstherapievorrichtung eine Aerosolerzeugungseinrichtung 1, eine Atemluftstromerzeugungseinrichtung 3 und einer Verneblungskammer 5, an die eine schlauchförmige Intubiereinrichtung 6 angeschlossen ist. Die Verneblungskammer 5 weist nicht nur einen sich verjüngenden und in die Intubiereinrichtung mündenden Bereich auf, sondern erlaubt auch die Durchmischung der zugeführten Atemluft 4 und der Flüssigkeitströpfchen 2, die von der Atemluftstromerzeugungseinrichtung 3 bzw. der Aerosolerzeugungseinrichtung 1 der Verneblungskammer 5 zugeführt werden. In einer besonders vorteilhaften Gestaltung, die in den Figuren 1 bis 5 gezeigt ist, wonach die Aerosolerzeugungseinrichtung 1 in der Verneblerkammer 5 angeordnet ist, stellt sich um die Aerosolerzeugungseinrichtung 1 aufgrund der Anordnung der Atemluftstromerzeugungseinrichtung 3 eine um die Aerosolerzeugungseinrichtung 1 herum verlaufende Atemluftströmung 4 ein. Dies führt vorteilhafterweise dazu, dass die Flüssigkeitströpfchen 2 von der Atemluftströmung mantelartig umschlossen werden und so eine Deposition in der Verneblungskammer 5 quasi ausgeschlossen ist, ohne dass eine gute Durchmischung der Flüssigkeitströpfchen der Atemluft 4 beeinträchtigt wird. Durch die sich verjüngende Zuführung der Atemluftströmung mit den darin geförderten Flüssigkeitströpfchen erfolgt eine optimale Zuführung des Flüssigkeitströpfchen/Atemluft-Gemisches in die Intubiereinrichtung. Trotz der möglicherweise gering erscheinenden Abmessungen, die für die Intubiereinrichtung erforderlich sind, tritt eine Deposition der Flüssigkeitströpfchen praktisch nicht auf, so dass das vernebelte Fluid, insbesondere ein vernebeltes Surfactant nahezu vollständig in die Lunge eines Frühgeborenen/Kleinkindes appliziert werden kann. Dazu trägt in erheblichem Maße auch die Gestaltung des Intubierschlauchs bei, dessen für die Intubation vorgesehenes Ende so gestaltet ist, dass eine Abgabe erst hinter den filternden Atemwegsbereichen (siehe oben) erfolgt.

In der vorangegangenen Beschreibung der Erfindung wurde insbesondere auf die Verabreichung eines Surfactants Bezug genommen. Jedoch ist der Beschreibung der Erfindung ebenfalls zu entnehmen, dass sich ein Inhalationstherapiesystem gemäß der Erfindung grundsätzlich für die inhalative Applikation von Medikamenten jedweder Art eignet, um Neugeborenen und Frühchen eine topische oder systemische Arzneitherapie mit dem erfindungsgemäßen Inhalationstherapiesystem zukommen zu lassen, dadurch gekennzeichnet, dass Körperfunktionen und/oder ein unnatürlicher oder krankhafter Zustand wieder in einen Normalzustand überführt und Leiden gelindert oder geheilt werden.

Die Arzneitherapie ist dadurch gekennzeichnet, dass mit dem erfindungsgemäßen Inhalationstherapiesystem besonders vorteilhaft Medikamente jedweder Art und Klasse aus tierischem, bakteriellem, menschlichem oder synthetischem Material inhalativ appliziert werden können, wie z.B.
- Lungensurfactant (wie z.B. Surfaxin),
- antientzündliche Mittel, wie Steroide (Ciclesonid, Fluticason),
- nicht-steroidale Entzündungshemmer (wie z.B. Ibuprofen, Celecoxib),
- Betamimetika (wie z.B. Indacaterol, Formoterol, Levalbuterol),
- Anticholinergika (wie z.B. Thiotropium, Glycopyrrolate, Ipratropium),
- Phosphodiesterasehemmer (wie z.B. Sildenafil, Vardenafil, Tadalafil),
- Endothelinantagonisten (wie z.B. Bosentan, Sixtasentan, Tezosentan),
- Leukotrienantagonisten (wie z.B. Montelukast),
- Diuretika (wie z.B. Furosemid, Amilorid),
- Immunmodulatoren (wie z.B. Cyclosporin, Mofetil, Sirolimus, Tacrolimus),
- Blutdrucksenkende Mittel (wie z.B. Statine, Sartane, Calcium- und Angiotension Antagonisten),
- Mucolytika (wie z.B. Dornase alpha, Bromhexin, Ambroxol, Acetycystein),
- Antibiotika verschiedenster Klassen, wie Chinolone, Makrolide, Cephalosporine, Aminoglycoside, Ketolide, Peptide und Proteine,
- Interferone,
- Immunglobuline,
- Prostine,
- Antimykotika,
- Antivirale Mittel,
- Heparin und Heparinoide,
- Cytostatika,
- körpereigene Substanzen, die durch einen Gendefekt wie z.B. Mucoviszidose oder Krankheitszustand nicht in ausreichender Menge im Körper verfügbar sind, wie z.B. Alpha-Antitrypsin, Interferone, Insulin, etc.

Diese Substanzen können in Form von Säuren oder Basen als pharmazeutisch gebräuchliche Salze oder Komplexe, Prodrugs oder ihrer optisch aktiven Antipoden, Stereoisomere, Enantiomere, allein oder in Kombinationen eingesetzt werden.

Besonders geeignete Medikamentenformulierungen sind dadurch gekennzeichnet, dass sie als wässrige Zubereitungen in Volumina von 0.3 bis 10 ml und besonders bevorzugt in Volumina von 0.5 bis 5 ml vernebelbar sind und mit dem erfindungsgemäßen Inhalationstherapiesystem ein Aerosol mit einem Medianen Massendurchmesser (MMD) von kleiner 5 *µ*m und besonders bevorzugt kleiner 3.5 *µ*m mit einer schmalbandigen Partikelverteilung erzeugbar ist, die durch eine geometrische Standardabweichung von kleiner 2 und besonders bevorzugt kleiner 1.6 ausgezeichnet ist, wodurch eine invitro Lungendosis in einem Cast-Modell > 20% und besonders bevorzugt > 25% erreicht wird, die über derjenigen herkömmlicher Inhalationstherapiesysteme mit Jet-Düsen-Vernebler liegt.

Die inhalative Medikamententherapie mit dem innovativen Inhalationstherapiesystem ist dadurch gekennzeichnet, dass damit respiratorische Krankheitsbilder, wie frühkindliche Lungenerkrankungen, Lungendistressysmptome, Asthma, obstruktive Bronchitis, bakterielle und nicht bakterielle Entzündungen, Husten, pulmonaler Bluthochdruck, parenchymale Erkrankungen, genetische Defekte wie z.B. Mucoviszidose, frühkindliche Diabetes, etc. bevorzugt behandelt werden können.

Aus einem anderen Blickwinkel kann das Inhalationstherapiesystem gemäß der Erfindung als geeignet angesehen werden für die verbesserte inhalative Applikation von Medikamenten mit einem innovativen Verneblerkonzept, das insbesondere auf die Bedürfnisse der Behandlung von Frühchen und auch normalen Kleinkindern abgestimmt ist.

Für die Erreichung einer besonders hohen Effizienz kann die Applikation von Medikamenten unter Anwendung verschiedener Applikationsstrategien erfolgen. Da die sich für den Einzelfall anbietende Applikationsstrategie von verschiedenen Randbedingungen, z.B. von dem zu applizierenden Medikament bzw. der zu applizierenden Medikamentenkombination, von den konstruktiven Details des Inhalationstherapiesystems, von der zu therapierenden Patientengruppe etc. abhängt, wird im Folgenden ein repräsentatives Beispiel erläutert, das einen durch eine Applikationsstrategie beeinflussbaren Bereich genauer darstellt. Vor dem Hintergrund experimenteller Ergebnisse, die aufzeigen, dass bei einer zu hohen Outputrate des Aerosolerzeugers die Effizienz niedriger liegt als bei einer verringerten Outputrate, bietet sich eine Applikationsstrategie an, die diesen Ergebnissen Rechnung trägt. Wesentlich für die hier beispielhaft erläuterte Applikationsstrategie ist eine Verringerung bzw. Anpassung der Outputrate des Aerosolerzeugers, wodurch die Applikationsstrategie zu einer erhöhten Effizienz führt. Die Verringerung/Anpassung der Outputrate wird durch wechselndes Ein/Aus-Schalten des Aerosolerzeugers auf besonders flexible Art und Weise erreicht, da durch Anpassung/Änderung der Ein/Aus-Schaltphasen die Outputrate in weiten Bereichen festgelegt (eingestellt) werden kann. Um die Wirksamkeit der Therapie nicht zu gefährden oder verzögert eintreten zu lassen, wird in einer speziellen Gestaltung der Applikationstherapie der Phase mit verringerter Outputrate eine Phase mit erhöhter Outputrate vorangestellt Dabei wird in Kauf genommen, dass die Effizienz während dieser vorgeschalteten Phase geringer ist und eine größere Medikamentenmenge eingesetzt werden muss als bei einer optimierten Outputrate. Durch die bewusst erhöhte Outputrate mit geringerer Effizienz wird aber erreicht, dass die angestrebte Wirkung des Medikaments zu einem gewünschten früheren Zeitpunkt einsetzt als dies bei einer Applikation bei verringerter Outputrate von Anfang an erzielbar wäre. Eine reduzierte Outputrate ist optimierend hinsichtlich der Wirkstoffausnutzung (=Effizienz der Applikation, minimierte Wirkstoffverluste im System) einsetzbar. Es ist offensichtlich, dass durch geeignete Applikationsstrategien sehr flexibel eine wirksame Therapie mit einer hohen Effizienz und Wirksamkeit verbunden werden kann.

Am Beispiel des bereits erwähnten Surfactants soll dieser Aspekt nochmals beispielhaft verdeutlicht werden. Wird in einer ersten Phase der Applikationsstrategie das Surfactant dem Frühgeborenen mit einer hohen Outputrate mit Hilfe des erfindungsgemäßen Inhalationstherapiesystems verabreicht, wird rasch die stabilisierende Wirkung des Surfactants auf die Lunge und einer Verbesserung der Lungenfunktion erreicht. Hat sich die Lungenfunktion des Frühgeborenen stabilisiert, kann in einer zweiten Phase der Applikationsstrategie die Outputrate mit dem Ziel verringert werden, den Einsatz des Surfactants nun hinsichtlich der Effizienz zu optimieren, wodurch eine bessere Ausnutzung des wertvollen Wirkstoffs, bei dem es sich nicht nur um das zuvor angesprochene Surfactant handeln muss, erreicht wird.

Ganz offensichtlich eignet sich ein Membranvernebler als Aerosolerzeuger in herausragender Weise für die Umsetzung der zuvor erläuterten Applikationsstrategie. Denn ein Membranvernebler lässt sich in besonders geeigneter Weise durch das Ansteuersignal an den die Membran in Schwingungen versetzenden Piezo-Schwinger ein- bzw. ausschalten. Der gepulste Betrieb, in dem sich Aerosolerzeugungsphasen und Ruhephase abwechseln, ist problemlos und sehr präzise zu realisieren. Auf diesem Weg ist eine Optimierung für jede sinnvolle/erforderliche Applikationsstrategie realisierbar und optimierbar.

## Patentansprüche

1. Inhalationstherapievorrichtung mit
einer Atemluftstromerzeugungseinrichtung (3) für die Erzeugung einer Atemluftströmung (4) und Förderung der Atemluftströmung (4) in eine Zuführleitung (32);
einer Aerosolerzeugungseinrichtung (1) mit einer Membran (13), einem Reservoir (12) für die Bevorratung einer Flüssigkeit und einem die Membran in Schwingungen versetzenden Piezo-Schwinger für die Vernebelung der aus dem Reservoir zugeführten Flüssigkeit und Bereitstellung von Flüssigkeitströpfchen (2);
einer Vernebelungskammer (5) mit einer Atemluftzuführöffnung (51) an der die Zuführleitung (32) angeschlossen ist, wobei sich die Atemluftströmung (4) und die Flüssigkeitströpfchen (2) in der Verneblungskammer (5) vermischen, und einem Bereich (52), der eine Austrittsöffnung (53) mit einem Stutzen (54) bildet, um das Flüssigkeitströpfchen/Atemluft-Gemisch aus der Verneblungskammer auszugeben;
einer Steuereinrichtung (14), die zur Steuerung der Aerosolerzeugungseinrichtung (1) und zum Ansteuern des Piezo-Schwingers ausgestaltet ist, um die Aerosolerzeugungseinrichtung (1) über ein Ansteuersignal an den Piezo-Schwinger wechselnd ein- bzw. auszuschalten, **dadurch gekennzeichnet, dass** die Inhalationstherapievorrichtung ausgestaltet ist EIN/AUS-Schaltphasen der Aerosolerzeugungseinrichtung (1) zu ändern und dadurch eine Outputrate der Aerosolerzeugungseinrichtung anzupassen bzw. einzustellen, so dass während einer Applikationstherapie, einer ersten Phase mit verringerter Outputrate eine Phase mit erhöhter Outputrate vorangestellt ist.

2. Inhalationstherapievorrichtung nach einem der vorangegangenen Ansprüche, **dadurch gekennzeichnet, dass** die Aerosolerzeugungseinrichtung (1) in der Verneblungskammer (5) angeordnet und bezogen auf den Bereich (52) so ausgerichtet ist, dass die Abgabe der Flüssigkeitströpfchen unmittelbar in die Verneblungskammer (5) auf den Bereich zu erfolgt, wobei die Aerosolerzeugungseinrichtung (1) von der Atemluftstromerzeugungseinrichtung zugeführten Atemluft (4) umströmt wird und dadurch die Flüssigkeitströpfchen (2) von der Atemluftströmung (4) mantelartig umschlossen werden und sich die Flüssigkeitströpfchen (2) und die Atemluftströmung (4) mischen.

3. Inhalationstherapievorrichtung nach einem der vorangegangenen Ansprüche, **dadurch gekennzeichnet, dass** die Aerosolerzeugungseinrichtung (1) zur Applikation von Medikamenten und bevorzugt Lungensurfactant ausgestaltet ist.

4. Inhalationstherapievorrichtung nach einem der Ansprüche 1 bis 2, **dadurch gekennzeichnet, dass** die Aerosolerzeugungseinrichtung (1) zur Applikation von Medikamenten und bevorzugt Antibiotika ausgestaltet ist.

5. Inhalationstherapievorrichtung nach einem der Ansprüche 1 bis 2, **dadurch gekennzeichnet, dass** die Aerosolerzeugungseinrichtung (1) zur Applikation von Medikamenten und bevorzugt antientzündliche Mitteln, wie Steroiden oder nicht-steroidalen Entzündungshemmern ausgestaltet ist.

6. Inhalationstherapievorrichtung nach einem der Ansprüche 1 bis 2, **dadurch gekennzeichnet, dass** die Aerosolerzeugungseinrichtung (1) zur Applikation von Medikamenten und bevorzugt Betamimetika ausgestaltet ist.

7. Inhalationstherapievorrichtung nach einem der Ansprüche 1 bis 2, **dadurch gekennzeichnet, dass** die Aerosolerzeugungseinrichtung (1) zur Applikation von Medikamenten und bevorzugt körpereigenen Substanzen, wie Alpha-Antitrypsin, Interferone und/oder Insulin ausgestaltet ist.

8. Inhalationstherapievorrichtung nach einem der Ansprüche 1 bis 2, **dadurch gekennzeichnet, dass** die Aerosolerzeugungseinrichtung (1) ausgestaltet ist Medikamentenformulierungen mit Volumina von 0,3 bis 10ml und bevorzugt Volumina von 0,3 bis 10ml zu vernebeln.

9. Inhalationstherapievorrichtung nach einem der vorangegangenen Ansprüche, **dadurch gekennzeichnet, dass** die Aerosolerzeugungseinrichtung (1) ausgestaltet ist ein Aerosol mit einem medianen Massendurchmesser (MMD) kleiner 5 µm und bevorzugt kleiner 3,5 µm zu erzeugen.

10. Inhalationstherapievorrichtung nach Anspruch 9, **dadurch gekennzeichnet, dass** die Aerosolerzeugungseinrichtung (1) ausgestaltet ist ein Aerosol mit einer Partikelverteilung mit einer Standardverteilung von kleiner 2 und bevorzugt eine Standardverteilung von kleiner 1,6 zu erzeugen.

## Claims

1. An inhalation therapy apparatus having
a respiratory air stream generating device (3) for generating a respiratory air stream (4) and conveying the respiratory air stream (4) into a delivery pipe (32);
an aerosol generating device (1) with a membrane (13), a reservoir (12) for storage of a liquid and a piezo-oscillator causing the membrane to vibrate for the nebulisation of the fluid conveyed out of the reservoir and preparation of liquid droplets (2);
a nebulisation chamber (5) with a respiratory air inlet opening (51) to which the delivery pipe (32) is connected, the respiratory air stream (4) and the liquid droplets (2) mixing in the nebulisation chamber (5), and a region (52) which forms an outlet opening (53) with a connection piece (54) in order to release the liquid droplet/respiratory air mixture from the nebulisation chamber;
a control device (14) which is designed to control the aerosol generating device (1) and to actuate the piezo-oscillator in order to alternately switch on and switch off the aerosol generating device (1) by means of an actuating signal sent to the piezo-oscillator, **characterised in that** the inhalation therapy apparatus is designed to change ON/OFF switching phases of the aerosol generating device (1) and so to adapt or set an output rate of the aerosol generating device so that during an application treatment, a phase with an increased output rate precedes a first phase with a reduced output rate.

2. The inhalation therapy apparatus according to the preceding claim, **characterised in that** the aerosol generating device (1) is disposed in the nebulisation chamber (5) and, in relation to the region (52), is aligned in such a way that dispensation of the liquid droplets is effected directly into the nebulisation chamber (5) onto the region, wherein the respiratory air (4) delivered by the respiratory air stream generating device flows round the aerosol generating device (1) and as a result the liquid droplets (2) are enveloped by the respiratory air stream (4) like a sheath and the liquid droplets (2) and the respiratory air stream (4) mix.

3. The inhalation therapy apparatus according to any of the preceding claims, **characterised in that** the aerosol generating device (1) is designed to apply medicines and preferably pulmonary surfactant.

4. The inhalation therapy apparatus according to either of Claims 1 to 2, **characterised in that** the aerosol generating device (1) is designed to apply medicines and preferably antibiotics.

5. The inhalation therapy apparatus according to either of Claims 1 to 2, **characterised in that** the aerosol generating device (1) is designed to apply medicines and preferably anti-inflammatories such as steroids or non-steroidal inflammation inhibitors.

6. The inhalation therapy apparatus according to either of Claims 1 to 2, **characterised in that** the aerosol generating device (1) is designed to apply medicines and preferably betamimetics.

7. The inhalation therapy apparatus according to either of Claims 1 to 2, **characterised in that** the aerosol generating device (1) is designed to apply medicines and preferably substances produced naturally by the body such as alpha antitrypsin, interferons and/or insulin.

8. The inhalation therapy apparatus according to either of Claims 1 to 2, **characterised in that** the aerosol generating device (1) is designed to nebulise medicine formulations with volumes of 0.3 to 10 ml and preferably volumes of 0.3 to 10 ml.

9. The inhalation therapy apparatus according to any of the preceding claims, **characterised in that** the aerosol generating device (1) is designed to generate an aerosol with a mass median diameter (MMD) of less than 5 µm and preferably less than 3.5 µm.

10. The inhalation therapy apparatus according to Claim 9, **characterised in that** the aerosol generating device (1) is designed to generate an aerosol with a particle distribution with a standard distribution of less than 2 and preferably a standard distribution of less than 1.6.

## Revendications

1. Dispositif de thérapie par inhalation, comprenant
un dispositif de génération de flux d'air respirable (3) pour générer un flux d'air respirable (4) et transporter le flux d'air respirable (4) dans un conduit d'amenée (32) ;
un dispositif de génération d'aérosol (1) avec une membrane (13), avec un réservoir (12) destiné au stockage d'un liquide et avec un oscillateur piézoélectrique faisant osciller la membrane et destiné à atomiser le liquide amené depuis le réservoir et à fournir des gouttelettes de liquide (2) ;
une chambre d'atomisation (5) avec une ouverture d'amenée d'air respirable (51) à laquelle est raccordé le conduit d'amenée (32), le flux d'air respirable (4) et les gouttelettes de liquide (2) se mélangeant dans la chambre d'atomisation (5), et avec une région (52) qui forme une ouverture de sortie (53) avec un raccord (54) pour délivrer le mélange gouttelettes de liquide et air respirable hors de la chambre d'atomisation ;
un dispositif de commande (14), qui est conçu pour commander le dispositif de génération d'aérosol (1) et pour commander l'oscillateur piézoélectrique afin de mettre en service et hors service, en alternance, le dispositif de génération d'aérosol (1) en envoyant un signal de commande à l'oscillateur piézoélectrique,
**caractérisé en ce que** le dispositif de thérapie par inhalation est conçu pour modifier des phases marche/arrêt du dispositif de génération d'aérosol (1) et pour adapter ou régler ainsi un taux de sortie du dispositif de génération d'aérosol de manière à, pendant une thérapie d'application, faire précéder une première phase présentant un taux de sortie réduit d'une phase présentant un taux de sortie accru.

2. Dispositif de thérapie par inhalation selon l'une quelconque des revendications précédentes, **caractérisé en ce que** le dispositif de génération d'aérosol (1) est disposé dans la chambre d'atomisation (5) et est orienté par rapport à la région (52) de telle manière que la délivrance des gouttelettes de liquide a lieu directement dans ladite région dans la chambre d'atomisation (5), le dispositif de génération d'aérosol (1) étant enveloppé par l'air respirable (4) conduit depuis le dispositif de génération de flux d'air respirable, les gouttelettes de liquide (2) étant de ce fait entourées par le flux d'air respirable (4) à la manière d'une enveloppe et les gouttelettes de liquide (2) se mélangeant au flux d'air respirable (4).

3. Dispositif de thérapie par inhalation selon l'une quelconque des revendications précédentes, **caractérisé en ce que** le dispositif de génération d'aérosol (1) est conçu pour l'application de médicaments et de préférence de surfactants pulmonaires.

4. Dispositif de thérapie par inhalation selon l'une quelconque des revendications 1 à 2, **caractérisé en ce que** le dispositif de génération d'aérosol (1) est conçu pour l'application de médicaments et de préférence d'antibiotiques.

5. Dispositif de thérapie par inhalation selon l'une quelconque des revendications 1 à 2, **caractérisé en ce que** le dispositif de génération d'aérosol (1) est conçu pour l'application de médicaments et de préférence d'agents antiinflammatoires, comme des stéroïdes ou des antiinflammatoires non stéroïdiens.

6. Dispositif de thérapie par inhalation selon l'une quelconque des revendications 1 à 2, **caractérisé en ce que** le dispositif de génération d'aérosol (1) est conçu pour l'application de médicaments et de préférence de bêtamimétiques.

7. Dispositif de thérapie par inhalation selon l'une quelconque des revendications 1 à 2, **caractérisé en ce que** le dispositif de génération d'aérosol (1) est conçu pour l'application de médicaments et de préférence de substances endogènes, comme l'alphaantitrypsine, l'interféron et/ou l'insuline.

8. Dispositif de thérapie par inhalation selon l'une quelconque des revendications 1 à 2, **caractérisé en ce que** le dispositif de génération d'aérosol (1) est conçu pour atomiser des formulations médicamenteuses ayant des volumes compris entre 0,3 et 10 ml et de préférence des volumes compris entre 0,3 et 10 ml.

9. Dispositif de thérapie par inhalation selon l'une quelconque des revendications précédentes, **caractérisé en ce que** le dispositif de génération d'aérosol (1) est conçu pour produire un aérosol avec un diamètre massique médian (MMD) inférieur à 5 µm et de préférence inférieur à 3,5 µm.

10. Dispositif de thérapie par inhalation selon la revendication 9, **caractérisé en ce que** le dispositif de génération d'aérosol (1) est conçu pour produire un aérosol avec une distribution de particules présentant une distribution standard inférieure à 2 et de préférence une distribution standard inférieure à 1,6.
